# NOUVEAU FASCICULE DE BREVET EUROPEEN

(11) **EP 1 374 842 B2**
(45) Date de publication et mention de la décision concernant l'opposition: **10.05.2017**
(45) Mention de la délivrance du brevet: 14.05.2008
(21) Numéro de dépôt: 03291531.6
(22) Date de dépôt: 23.06.2003
(51) Int. Cl.: A61K 8/22, A61K 8/25, A61K 8/41, A61Q 5/10

(54) **Composition pour la teinture d'oxydation des fibres kératiniques humaines**
Zusammensetzung zur Oxidationsfärbung von keratinischen Fasern bei Menschen
Composition for the oxidative dyeing of human keratin fibres

(30) Priorité: 26.06.2002 FR 0207938
(43) Date de publication de la demande: 02.01.2004
(73) Titulaire: L'Oréal, 75008 Paris (FR)
(72) Inventeur: Cottard, Francois, 92400 Courbevoie (FR); Rondeau, Christine, 78500 Sartrouville (FR)
(74) Mandataire: Wattremez, Catherine

(56) Documents cités:
- WO-A1-00/76469
- DE-A- 4 135 760
- DE-A- 19 962 869
- DE-A1- 19 527 121
- DE-A1- 19 824 685
- DE-C1- 19 713 698
- GB-A- 2 033 939
- D.F. WILLIAMS AND W.H. SCHMITT ED.: "Chemistry and technology of the cosmetics and toiletries industry, 2nd edition, 1996" , BLACKIE ACADEMIC AND PROFESSIONAL XP002255267 229190 * page 93-97 *
- R.C. PEPE ET AL. ED.: "International cosmetic ingredient dictionary and handbook, 9th ed., 2002, vol.2" , THE COSMETIC, TOILETRY AND FRAGRANCE ASSOCIATION , USA XP002237676 271536 * page 1578 *
- J. FALBE, PROF. DR. ET AL.: 'Römpp Chemie Lexikon', vol. 9, 1995, GEORG THIEME VERLAG, STUTTGART - NEW YORK page 2936

## Description

L'invention a pour objet une composition pour la teinture d'oxydation des fibres kératiniques humaines et en particulier des cheveux comprenant, dans un milieu cosmétiquement acceptable à base d'eau et à pH basique, au moins un colorant d'oxydation et un agent alcalinisant comprenant au moins un métasilicate alcalin, alcalino-terreux ou d'ammonium et au moins une alcanolamine avec un rapport pondéral méthasilicate anhydre/alcanolamine compris entre 0,2 et 100, ainsi que le procédé de teinture mettant en oeuvre cette composition.

Il est connu de teindre les fibres kératiniques humaines et en particulier les cheveux avec des compositions tinctoriales contenant des colorants d'oxydation. Les colorants d'oxydation comprennent les précurseurs de colorant d'oxydation et les coupleurs.
Les précurseurs de colorants d'oxydation, appelés généralement bases d'oxydation, sont des composés incolores ou faiblement colorés qui, associés à des produits oxydants, peuvent donner naissance par un processus de condensation oxydative à des composés colorés et colorants. Ce sont en particulier des ortho ou paraphénylènediamines, des ortho ou paraaminophénols, ou des bases hétérocycliques.

Les nuances obtenues avec ces bases d'oxydation peuvent être modifiées par association desdites bases avec des coupleurs ou modificateurs de coloration, les coupleurs étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

Le procédé de teinture d'oxydation consiste à appliquer sur les fibres, des bases d'oxydation ou un mélange de bases d'oxydation et de coupleurs avec un agent oxydant, le plus souvent de l'eau oxygénée, à laisser pauser, puis à rincer les fibres. L'application réalisée généralement à pH basique permet d'obtenir une teinture et simultanément un éclaircissement de la fibre ce qui se traduit en pratique par la possibilité d'obtenir une coloration finale plus claire que la couleur d'origine. En outre, l'éclaircissement de la fibre a pour effet avantageux d'engendrer une couleur unie dans le cas des cheveux gris, et dans le cas de cheveux naturellement pigmentés, de faire ressortir la couleur, c'est à dire de la rendre plus visible.

L'éclaircissement des cheveux est évalué par la hauteur de ton qui caractérise le degré ou le niveau d'éclaircissement. La notion de « ton » repose sur la classification des nuances naturelles, un ton séparant chaque nuance de celle qui la suit ou la précède immédiatement. Cette définition et la classification des nuances naturelles est bien connue des professionnels de la coiffure et publiée dans l'ouvrage « Sciences des traitements capillaires » de Charles ZVIAK 1988, Ed. Masson, pp.215 et 278.
Les hauteurs de ton s'échelonnent de 1 (noir) à 10 (blond clair clair), une unité correspondant à un ton ; plus le chiffre est élevé et plus la nuance est claire.

La technologie de teinture d'oxydation éclaircissante qui doit permettre d'obtenir un éclaircissement suffisant de la fibre et une couverture des cheveux blancs à 100% a jusqu'ici impliqué d'utiliser soit de l'ammoniaque, soit de la monoéthanolamine, soit un mélange de monoéthanolamine et d'ammoniaque, comme agent alcalinisant.
Or, comme chacun sait, l'ammoniaque présente le gros inconvénient de dégager une odeur désagréable au cours de l'application de la teinture.
La monoéthanolamine, si elle est utilisée à de fortes concentrations, provoque parfois des irritations du cuir chevelu sous forme de picotements.

Le document DE 199 62 869 A1 divulgue l'utilisation de composés siliciés pour diminuer la dégradation des cheveux lors de processus oxydants.

Le document DE 41 35 760 A1 divulgue une composition de teinture oxydante avec du métasilicate de soude.

Le document GB 2 033 939 divulgue des compositions pour éclaircir la teinte des cheveux comprenant en mélange du métasilicate de soude et de la monoéthanolamine.

Voici maintenant qu'après d'importantes recherches menées sur la question, la Demanderesse vient de découvrir qu'il est possible de diminuer l'odeur désagréable et les risques d'irritation du cuir chevelu desdites teintures tout en obtenant le niveau d'éclaircissement souhaité et des colorations intenses dans des nuances variées, en utilisant à titre d'agent alcalinisant, un mélange d'au moins un métasilicate de métal alcalin ou alcalino-terreux ou d'ammonium et d'au moins une alcanolamine avec un rapport pondéral méthasilicate anhydre/alcanolamine compris entre 0,2 et 100.

Cette découverte est à la base de la présente invention.

L'invention a donc pour premier objet une composition pour la teinture d'oxydation des fibres kératiniques humaines et plus particulièrement des cheveux comprenant, dans un milieu cosmétiquement acceptable à base d'eau, et à pH basique, au moins un colorant d'oxydation et un agent alcalinisant, caractérisée en ce que l'agent alcalinisant est une association d'au moins un métasilicate choisi dans le groupe formé par les métasilicates alcalins, alcalino-terreux ou d'ammonium et d'au moins une alcanolamine avec un rapport pondéral méthasilicate anhydre/alcanolamine compris entre 0,2 et 100.

La composition tinctoriale conforme à l'invention permet de formuler des produits moins odorants et moins irritants et de réduire notamment le taux d'alcanolamine classiquement utilisé avec un parfait maintien des propriétés tinctoriales.

L'invention a également pour objet un procédé de teinture d'oxydation des fibres kératiniques humaines et plus particulièrement des cheveux mettant en oeuvre ladite composition.

Elle a pour autre objet une composition prête à l'emploi pour la teinture d'oxydation des fibres kératiniques humaines et plus particulièrement des cheveux comprenant le mélange d'une composition décrite ci-dessus et d'une composition oxydante.
Par "composition prête à l'emploi" on entend, au sens de l'invention, la composition destinée à être appliquée telle quelle sur les fibres kératiniques, c'est à dire qu'elle peut être stockée telle quelle avant utilisation ou résulter du mélange extemporané de deux compositions.

### Agent alcalinisant

L'association selon l'invention d'au moins un métasilicate de métal alcalin ou alcalino-terreux ou d'ammonium et d'au moins une alcanolamine, utilisée à titre d'agent alcalinisant doit permettre d'ajuster le pH de la composition colorante de la présente invention de 7,2 à 13 et de préférence de 8,5 à 11,5.
Selon la présente invention, ladite association comprend en matière active:
- de 0,1 à 6% en poids environ d'un ou plusieurs métasilicates, de préférence de 0,5 à 5% et plus particulièrement de 1 à 3%, et,
- de 0,1 à 8% en poids environ d'une ou plusieurs alcanolamines, de préférence de 0,5 à 6 et plus particulièrement encore de 1 à 5,5%,
par rapport au poids total de la composition.

Les métasilicates utilisables selon la présente invention peuvent être choisis parmi les métasilicates de sodium, de potassium ou d'ammonium. De préférence, on utilise le métasilicate de sodium.

Le métasilicate de sodium [Na₂SiO₃] est un composé anhydre mais il peut également se présenter sous ses formes hydratées à 5 ou 9 molécules d'eau.

Les alcanolamines peuvent être choisies dans le groupe formé par la monoéthanolamine, la triéthanolamine, la monoisopropanolamine, la diisopropanolamine, la N-diméthylaminoéthanolamine, le 2-amino-2-méthyl-1-propanol, la trüsopropanolamine, le 2-amino-2-méthyl-1,3-propanediol, le 3-amino-1,2-propanediol, le 3-diméthylamino-1,2-propanediol, le tris-hydroxyméthylaminométhane.
De préférence, on utilise la monoéthanolaamine.

Le rapport pondéral métasilicate anhydre / alcanolamine est compris entre 0,2 et 100, plus particulièrement entre 0,2 et 10, et de préférence entre 0,2 et 2.

### Milieu

Le milieu cosmétiquement acceptable pour la teinture conforme à l'invention est constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ; le glycérol; les polyols et éthers de polyols comme le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.
Les solvants peuvent être présents dans des proportions allant de préférence de 1 à 40 % en poids environ par rapport au poids total de la composition tinctoriale, et encore plus préférentiellement de 5 à 30 % en poids environ.

### Colorants d'oxydation

Les colorants d'oxydation utilisables selon l'invention sont choisis parmi les bases d'oxydation et/ou les coupleurs.
De préférence les compositions selon l'invention contiennent au moins une base d'oxydation.
Les bases d'oxydation sont choisies parmi celles classiquement connues en teinture d'oxydation, et parmi lesquelles on peut notamment citer les ortho- et para-phénylènediamines, les bases doubles, les ortho- et para- aminophénols, les bases hétérocycliques suivantes ainsi que leurs sels d'addition avec un acide.
On peut notamment citer :
- (**I**) les paraphénylènediamines de formule (I) suivante et leurs sels d'addition avec un acide : 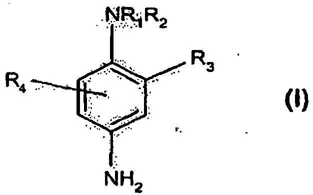 dans laquelle :
   R₁
      représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
   R₂
      représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
   R₁ et R₂
      peuvent également former avec l'atome d'azote qui les porte un hétérocycle azoté à 5 ou 6 chaînons éventuellement substitué par un ou plusieurs groupements alkyle, hydroxy ou uréido;
   R₃
      représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
   R₄
      représente un atome d'hydrogène, d'halogène ou un radical alkyle en C₁-C₄.
   Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les paraphénylènediamines de formule (I) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthy),β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-p-acétylaminoéthyloxy-paraphénylènediamine, la N-(β-méthoxyéthyl)-paraphénylènediamine, la 2-méthyl-1-N-β-hydroxyéthyl-paraphénylènediamine, la N-(4-aminophényl)-3-hydroxy-pyrrolidine, et leurs sels d'addition avec un acide.
   Parmi les paraphénylènediamines de formule (I) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxy-éthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.
- (**II**) Selon l'invention, on entend par bases doubles, les composés comportant au moins deux noyaux aromatiques sur lesquels sont portés des groupements amino et/ou hydroxyle.
   Parmi les bases doubles utilisables à titre de bases d'oxydation dans les compositions tinctoriales conformes à l'invention, on peut notamment citer les composés répondant à la formule (II) suivante, et leurs sels d'addition avec un acide : 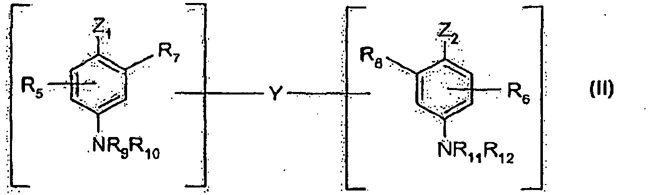 dans laquelle :
   ∘ Z₁ et Z₂, identiques ou différents, représentent un radical hydroxyle ou -NH₂ pouvant être substitué par un radical alkyle en C₁-C₄ ou par un bras de liaison Y ;
   ∘ le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
   ∘ R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
   ∘ R₇, R₈, R₉, R₁₀, R₁₁ et R₁₂, identiques ou différents, représentent un atome d'hydrogène, un bras de liaison Y ou un radical alkyle en C₁-C₄ ;
   étant entendu que les composés de formule (II) ne comportent qu'un seul bras de liaison Y par molécule.
   Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les bases doubles de formules (II) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide. Parmi ces bases doubles de formule (II), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.
- (**III**) les para-aminophénols répondant à la formule (**III**) suivante, et leurs sels d'addition avec un acide : 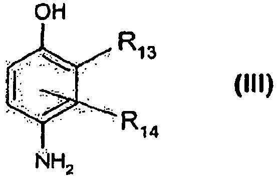 dans laquelle :
   **R₁₃** représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
   **R₁₄** représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).
   Parmi les para-aminophénols de formule (III) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.
- (**IV**) les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.
- (**V**) parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

Selon la présente invention, les bases d'oxydation représentent de préférence de 0,0005 à 12% en poids environ du poids total de la composition et encore plus préférentiellement de 0,005 à 8% en poids environ de ce poids.

Les coupleurs utilisables dans la composition de teinture selon l'invention sont ceux classiquement utilisés dans les compositions de teinture d'oxydation, c'est-à-dire les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 2-chloro-3-amino-6-méthyl-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy-benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Lorsqu'ils sont présents, ces coupleurs représentent de préférence de 0,0001 à 10% en poids environ du poids total de la composition, et encore plus préférentiellement de 0,005 à 5% en poids environ.

D'une manière générale, les sels d'addition avec un acide des bases d'oxydation et coupleurs sont notamment choisis parmi les chlorhydrates, les bromhydrates, les sulfates et les tartrates, les lactates et les acétates.

La composition selon l'invention peut encore contenir, en plus des colorants d'oxydation définis ci-dessus, des colorants directs pour enrichir les nuances en reflets. Ces colorants directs peuvent notamment alors être choisis parmi les colorants nitrés, azoïques ou anthraquinoniques, neutres, cationiques ou anioniques dans la proportion pondérale d'environ 0,001 à 20% et de préférence de 0,01 à 10% du poids total de la composition.

### Adjuvants

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des alcools gras, des acides gras, des polymères anioniques, cationiques, non-ioniques, amphotères, zwittérioniques ou leurs mélanges, des agents épaississants minéraux ou organiques ou des polymères épaississants tels que par exemple des gommes de guar non-ioniques, des polymères associatifs comportant au moins un motif hydrophile et au moins une chaîne grasse et de nature non ionique, anionique, cationique ou amphotère, des agents antioxydants ou réducteurs, des agents de pénétration, des agents séquestrants tels que l'EDTA et l'acide étidronique, des filtres UV, des cires, des parfums, des tampons, des agents dispersants, des agents de conditionnement tels que par exemple des silicones volatiles ou non volatiles, modifiées ou non modifiées, des agents filmogènes, des agents nacrants, es agents conservateurs, des céramides, des pseudocéramides, des huiles végétales, minérales ou de synthèse, des vitamines ou provitamines comme le panthénol, des opacifiants, etc....

De préférence, la composition tinctoriale de l'invention contient au moins un polymère cationique dans la proportion d'environ 0,05 à 10% en poids et au moins un tensioactif de préférence non ionique dans la proportion de 0,1 à 20% en poids.
De préférence elle contient également au moins un polymère épaississant choisi de préférence parmi les polymères associatifs dans la proportion d'environ 0,05 à 10% en poids.

Les agents réducteurs ou antioxydants peuvent être choisis en particulier parmi le sulfite de sodium, l'acide thioglycolique et l'acide thiolactique ainsi que leurs sels d'ammonium, le bisulfite de sodium, l'acide déhydroascorbique, l'hydroquinone, la 2-méthyl-hydroquinone, la ter-butyl-hydroquinone et l'acide homogentisique, et ils sont alors généralement présents dans des quantités allant d'environ 0,05 à 3% en poids par rapport au poids total de la composition.

La composition tinctoriale de l'invention peut également comprendre de l'ammoniaque. Plus particulièrement, la teneur en ammoniaque dans la composition tinctoriale est d'au plus 2 % en poids (solution aqueuse d'ammoniac à 20 % en poids).

Bien entendu, l'homme de l'art veillera à choisir ce ou ces éventuels composés complémentaires de manière telle que les propriétés avantageuses attachées intrinsèquement à la composition tinctoriale conforme à l'invention ne soient pas, ou substantiellement pas, altérées par la ou les adjonctions envisagées

Le procédé de teinture selon l'invention comprend les étapes suivantes : on mélange, au moment de l'emploi, une composition colorante telle que décrite ci-dessus et comprenant donc, dans un milieu cosmétiquement acceptable à base d'eau et à pH basique allant de 7,2 à 13, au moins un colorant d'oxydation et une association de métasilicate(s) et d'alcanolamine(s) selon l'invention, avec une composition oxydante, on applique ensuite le mélange obtenu sur les fibres kératiniques, on laisse poser pendant 3 à 50 minutes environ, de préférence 5 à 30 minutes environ, après quoi on rince, on lave au shampooing, on rince à nouveau et on sèche.

La composition colorante avant mélange avec l'oxydant peut se présenter sous des formes diverses, telles que liquide, crème, gel, éventuellement pressurisées ou sous toute autre forme appropriée pour réaliser après mélange une teinture des fibres kératiniques humaines et notamment des cheveux.

### Oxydant

Dans la composition oxydante, l'agent oxydant est choisi parmi le peroxyde d'hydrogène et les composés susceptibles de libérer du peroxyde d'hydrogène in situ, les enzymes d'oxydoréduction telles que les laccases, les peroxydases et les oxydoréductases à 2 électrons (telles que l'uricase), le cas échéant en présence de leur donneur ou cofacteur respectif.
L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.

Selon un mode de réalisation particulier de l'invention, le rapport pondéral composition colorante / composition oxydante est compris entre 2/1 et 1/6, de préférence entre 1/1 et 1/3.

Les exemples qui suivent sont destinés à illustrer l'invention sans pour autant présenter un caractère limitatif.

### EXEMPLES 1 - 3

On a préparé les compositions tinctoriales suivantes :
(teneurs exprimées en grammes de **matière active**)

| | Exemple | Exemple | Exemple |
|---|---|---|---|
| | 1 | 2 | 3 |
| Paraphénylènediamine | 0,24 | 0,24 | 0,24 |
| Para-aminophénol | 0,44 | 0,44 | 0,44 |
| 2-amino-phénol | 0,028 | 0,028 | 0,028 |
| 1,3-dihydroxy-benzène | 0,192 | 0,192 | 0,192 |
| 3-amino-phénol | 0,019 | 0,019 | 0,019 |
| 5-N-(β-hydroxyéthyl)-amino-2-méthyl-phénol | 0,021 | 0,021 | 0,021 |
| 1,3-dihydroxy-2-méthyl-benzène | 0,055 | 0,055 | 0,055 |
| métasilicate de sodium anhydre | 2 | 2 | 2 |
| Monoéthanolamine | 5,45 | 5,45 | 5,45 |
| Réducteur, antioxydant, séquestrant, parfum | q.s_{.} | q.s. | q.s |
| Propylène glycol | 10 | 10 | 10 |
| Polymère anionique : acide polyacrylique réticulé | 0,4 | 0,4 | 0,4 |
| Polymère amphotère: Polyquaternium 22 (Nom C.T.F.A.) Merquat 280 vendu par la société ONDEO | 1,5 | 1,5 | |
| Polymère cationique : Polyquaternium 6 (Nom C.T.F.A.) Merquat 100 vendu par la société ONDEO | | | 2,8 |
| Polymère cationique : Hexadimethrine Chloride (Nom C.T.F.A.) Mexomère PO vendu par la société CHIMEX | 3 | 3 | |
| Tensioactif anionique : laurytsulfate de sodium en poudre | 3 | | |
| Tensioactif non ionique: alcool laurique oxyéthyléné à 12 moles d'oxyde d'éthylène | | 7,5 | 7,5 |
| Tensioactif non ionique : alcool oléocétylique oxyéthyléné à 30 moles d'oxyde d'éthylène | | 4 | 4 |
| Tensioactif non ionique : alcool décylique oxyéthyléné à 3 moles d'oxyde d'éthylène | 10 | 10 | 10 |
| Tensioactif non ionique : alcool décylique oxyéthyléné à 5 moles d'oxyde d'éthylène | 8 | | |
| Acide laurique | 2,5 | 2,5 | 2,5 |
| Alcool cétylstéarylique 50/50 | 11,5 | 11,5 | 11,5 |
| Agent nacrant : Silice pyrogénée hydrophobe | 1,2 | 1,2 | 1,2 |
| Agent nacrant : Monostéarate de glycérol | 2 | 2 | 2 |
| Eau déminéralisée q.s.p | 100 | 100 | 100 |

Au moment de l'emploi, on a mélangé poids pour poids, chaque composition tinctoriale décrite ci-dessus avec une solution de peroxyde d'hydrogène à 20 volumes (6% en poids).
Les mélanges ainsi réalisés ont été appliqués pendant 30 minutes sur des mèches de cheveux gris naturels ou permanentés à 90 % de blancs. Les mèches ont ensuite été rincées, lavées avec un shampooing standard, rincées à nouveau puis séchées. On a pu constater que ces mélanges étaient beaucoup moins odorants que ceux de l'art antérieur avec des qualités d'application satisfaisantes.

Les cheveux ont été teints dans une nuance blond doré pour chacun des exemples 1 à 3.
Par rapport à des compositions de l'art antérieur identiques sauf qu'elles ne contiennent pas de métasilicate de sodium et présentent une teneur en monoéthanolamine bien plus élevée (de l'ordre de 10 % en poids de la composition tinctoriale), les performances tinctoriales des compositions 1 à 3 ont été conservées.

## Revendications

1. Composition pour la teinture d'oxydation des fibres kératiniques humaines et plus particulièrement des cheveux, comprenant, dans un milieu cosmétiquement acceptable à base d'eau, et à pH basique, au moins un colorant d'oxydation et un agent alcalinisant, **caractérisée en ce que** l'agent alcalinisant est une association d'au moins un métasilicate choisi dans le groupe formé par les métasilicates alcalins, alcalino-terreux ou d'ammonium, et d'au moins une alcanolamine avec un rapport pondéral métasilicate anhydre / alcanolamine compris entre 0,2 et 100.

2. Composition selon la revendication 1, **caractérisée en ce que** le métasilicate est un métasilicate de sodium.

3. Composition selon l'une quelconque des revendications précédentes, **caractérisée en ce que** les alcanolamines sont choisies dans le groupe formé par la monoéthanolamine, la triéthanolamine, la monoisopropanolamine, la diisopropanolamine, la N-diméthylaminoéthanolamine, le 2-amino-2-méthyl-1-propanol, la triisopropanolamine, le 2-amino-2-méthyl-1,3-propanediol, le 3-amino-1,2-propanediol, le 3-diméthylamino-1,2-propanediol, le tris-hydroxyméthylaminométhane.

4. Composition selon la revendication 3, **caractérisée en ce que** l'alcanolamine est la monoéthanolamine.

5. Composition selon l'une quelconque des revendications précédentes, **caractérisée en ce que** l'agent alcalinisant comprend en matière active, de 0,1 à 6% en poids de métasilicate par rapport au poids total de la composition.

6. Composition selon la revendication 5, **caractérisée en ce que** l'agent alcalinisant comprend en matière active, de 0,5 à 5% en poids de métasilicate par rapport au poids total de la composition.

7. Composition selon la revendication 6, **caractérisée en ce que** l'agent alcalinisant comprend en matière active, de 1 à 3% en poids de métasilicate par rapport au poids total de la composition.

8. Composition selon l'une quelconque des revendications précédentes, **caractérisée en ce que** l'agent alcalinisant comprend en matière active, de 0,1 à 8% en poids d'alcanolamine, par rapport au poids total de la composition.

9. Composition selon la revendication 8, **caractérisée en ce** l'agent alcalinisant comprend en matière active, de 0,5 à 6% en poids d'alcanolamine, par rapport au poids total de la composition.

10. Composition selon la revendication 9, **caractérisée en ce que** l'agent alcalinisant comprend en matière active, de 1 à 5,5% en poids d'alcanolamine, par rapport au poids total de la composition.

11. Composition selon l'une quelconque des revendications précédentes, **caractérisée en ce que** son pH est de 7,2 à 13.

12. Composition selon la revendication 11, **caractérisée en ce que** son pH est de 8,5 à 11,5.

13. Composition selon l'une quelconque des revendications précédentes, **caractérisée en ce que** le ou les colorants d'oxydation sont choisis parmi les bases d'oxydation et les coupleurs.

14. Composition selon la revendication 13, **caractérisée en ce qu**'elle comprend au moins une base d'oxydation.

15. Composition selon la revendication 14, **caractérisée en ce que** les bases d'oxydation sont choisies dans le groupe formé par les ortho- et para-phénylènediamines, les bases doubles, les ortho- et para- aminophénols, les bases hétérocycliques et leurs sels d'addition avec un acide.

16. Composition selon la revendication 13, **caractérisée en ce que** les coupleurs sont choisis dans le groupe formé par les méta-aminophénols, les méta-phénylènediamines, les métadiphénols, les naphtols, les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines, et leurs sels d'addition avec un acide.

17. Composition selon l'une quelconque des revendications 13 à 16, **caractérisé en ce que** les sels d'addition avec un acide des bases d'oxydation et des coupleurs sont choisis parmi les chlorhydrates, les bromhydrates, les sulfates et les tartrates, les lactates et les acétates.

18. Composition selon l'une quelconque des revendications 13-15 et 17, **caractérisée en ce que** le ou les bases d'oxydation sont présentes à une concentration allant de 0,0005 à 12% en poids par rapport au poids total de la composition.

19. Composition selon l'une quelconque des revendications 13, 16 et 17, **caractérisée en ce que** le ou les coupleurs sont présents à une concentration comprise entre 0,0001 et 10% en poids par rapport au poids total de la composition.

20. Composition selon l'une quelconque des revendications précédentes, **caractérisée en ce que** le milieu cosmétiquement acceptable à base d'eau comprend au moins un solvant organique.

21. Composition selon la revendication 20, **caractérisée en ce que** le ou les solvants organiques sont présents dans des proportions allant de 1 à 40 % en poids par rapport au poids total de la composition.

22. Composition selon l'une quelconque des revendications précédentes, **caractérisée en ce qu**'elle contient au moins un polymère cationique dans la proportion de 0,05 à 10% en poids et au moins un tensioactif non ionique dans la proportion de 0,1 à 20% en poids par rapport au poids total de la composition.

23. Procédé de teinture des fibres kératiniques humaines et en particulier des cheveux **caractérisé en ce qu**'on mélange, au moment de l'emploi, une composition colorante telle que décrite à l'une quelconque des revendications 1 à 22 avec une composition oxydante, qu'on applique le mélange obtenu sur les fibres, on laisse poser pendant 3 à 50 minutes, de préférence 5 à 30 minutes, après quoi on rince, on lave au shampooing, on rince à nouveau et on sèche, ladite composition oxydante comprenant du peroxyde d'hydrogène ou un composé susceptible de libérer du peroxyde d'hydrogène in situ, ou une enzyme d'oxydoréduction.

## Patentansprüche

1. Zusammensetzung zum oxidativen Färben von menschlichen Keratinfasern und insbesondere Haaren, die in einem kosmetisch akzeptablen Medium auf der Basis von Wasser bei einem basischen pH-Wert mindestens einen oxidationsfarbstoff und ein Alkalisierungsmittel enthält, **dadurch gekennzeichnet, dass** es sich bei dem Alkalisierungsmittel um eine Kombination aus mindestens einem Metasilicat, das unter den Alkalimetasilicaten, Erdalkalimetasilicaten oder Ammoniummetasilicaten ausgewählt ist, und mindestens einem Alkanolamin handelt, wobei das Gewichtsverhältnis von Metasilicat zu Alkanolamin zwischen 0,2 und 100 liegt.

2. Zusammensetzung nach Anspruch 1, **dadurch gekennzeichnet, dass** das Metasilicat ein Natriummetasilicat ist.

3. Zusammensetzung, nach einem der vorhergehenden Ansprüche, **dadurch gekennzeichnet, dass** die Alkanolamine unter Monoethanolamin, Triethanolamin, Monoisopropanolamin, Diisopropanolamin, N-Dimethylaminoethanolamin, 2-Amino-2-methyl-1-propanol, Triisopropanolamin, 2-Amino-2-methyl-1,3-propandiol, 3-Amino-1,2-propandiol, 3-Dimethylamino-1,2-propandiol und Tris-hydroxymethylaminomethan ausgewählt sind.

4. Zusammensetzung nach Anspruch 3, **dadurch gekennzeichnet, dass** das Alkanolamin das Monoethanolamin ist.

5. Zusammensetzung nach einem der vorhergehenden Ansprüche, **dadurch gekennzeichnet, dass** das Alkalisierungsmittel als wirksame Substanz 0,1 bis 6 Gew. -% Metasilicat, bezogen auf das Gesamtgewicht der Zusammensetzung, umfasst.

6. Zusammensetzung nach Anspruch 5, **dadurch gekennzeichnet, dass** das Alkalisierungsmittel als wirksame Substanz 0,5 bis 5 Gew.-% Metasilicat, bezogen auf das Gesamtgewicht der Zusammensetzung, umfasst.

7. Zusammensetzung nach Anspruch 6, **dadurch gekennzeichnet, dass** das Alkalisierungsmittel als wirksame Substanz 1 bis 3 Gew.-% Metasilicat, bezogen auf das Gesamtgewicht der Zusammensetzung, umfasst.

8. Zusammensetzung nach einem der vorhergehenden Ansprüche, **dadurch gekennzeichnet, dass** das Alkalisierungsmittel als wirksame Substanz 0,1 bis 8 Gew.-% Alkanolamin, bezogen auf das Gesamtgewicht der Zusammensetzung, umfasst.

9. Zusammensetzung nach Anspruch 8, **dadurch gekennzeichnet, dass** das Alkalisierungsmittel als wirksame Substanz 0,5 bis 6 Ges.-% Alkanolamin, bezogen auf das Gesamtgewicht der Zusammensetzung, umfasst.

10. Zusammensetzung nach Anspruch 9, **dadurch gekennzeichnet, dass** das Alkalisierungsmittel als wirksame Substanz 1 bis 5,5 Gew.-% Alkanolamin, bezogen auf das Gesamtgewicht der Zusammensetzung, umfasst.

11. Zusammensetzung nach einem der vorhergehenden Ansprüche, **dadurch gekennzeichnet, dass** ihr pH-Wert im Bereich von 7,2 bis 13 liegt.

12. Zusammensetzung nach Anspruch 11, **dadurch gekennzeichnet, dass** ihr pH-Wert im Bereich von 8,5 bis 11,5 liegt.

13. Zusammensetzung nach einem der vorhergehenden Ansprüche, **dadurch gekennzeichnet, dass** der oder die Oxidationsfarbstoffe unter den Oxidationsbasen und Kupplern ausgewählt sind.

14. Zusammensetzung nach Anspruch 13, **dadurch gekennzeichnet, dass** sie mindestens eine Oxidationsbase enthält.

15. Zusammensetzung nach Anspruch 14, **dadurch gekennzeichnet, dass** die Oxidationsbasen unter den ortho- und para-Phenylendiaminen, Doppelbasen, ortho- und para-Aminophenolen, heterocyclischen Basen und deren Additionssalzen mit einer Säure ausgewählt sind.

16. Zusammensetzung nach Anspruch 13, **dadurch gekennzeichnet, dass** die Kuppler unter den meta-Aminophenolen, meta-Phenylendiaminen, meta-Dihydroxybenzolen, Naphtholen, Indolderivaten, Indolinderivaten, Sesamol und seinen Derivaten, Pyridinderivaten, Pyrazolotriazolderivaten, Pyrazolonen, Indazolen, Benzimidazolen, Benzothiazolen, Benzoxazolen, 1,3-Benzodioxolen, Chinolinen und deren Additionssalzen mit einer Säure ausgewählt sind.

17. Zusammensetzung nach einem der Ansprüche 13 bis 16, **dadurch gekennzeichnet, dass** die Additionssalze der Oxidationsbasen und Kuppler mit einer Säure unter den Hydrochloriden, Hydrobromiden, Sulfaten, Tartraten, Lactaten und Acetaten ausgewählt sind.

18. Zusammensetzung nach einem der Ansprüche 13 bis 15 und 17, **dadurch gekennzeichnet, dass** die Oxidationsbase(n) in einer Konzentration von 0,0005 bis 12 Gew.-%, bezogen auf das Gesamtgewicht der Zusammensetzung, enthalten sind.

19. Zusammensetzung nach einem der Ansprüche 13, 16 und 17, **dadurch gekennzeichnet, dass** der oder die Kuppler in einer Konzentration von 0,0001 bis 10 Gew.-%, bezogen auf das Gesamtgewicht der Zusammensetzung, enthalten sind.

20. Zusammensetzung nach einem der vorhergehenden Ansprüche, **dadurch gekennzeichnet, dass** das kosmetisch akzeptable Medium auf der Basis von Wasser mindestens ein organisches Lösungsmittel enthält.

21. Zusammensetzung nach Anspruch 20, **dadurch gekennzeichnet, dass** das oder die organischen Lösungsmittel in Mengenanteilen von 1 bis 40 Gew.-%, bezogen auf das Gesamtgewicht der Zusammensetzung, enthalten sind.

22. Zusammensetzung nach einem der vorhergehenden Ansprüche, **dadurch gekennzeichnet, dass** sie mindestens ein kationisches Polymer in einer Menge von 0,05 bis 10 Gew.-% und mindestens einen nichtionischen grenzflächenaktiven Stoff in einer Menge von 0,1 bis 20 Ges.-%, bezogen auf das Gesamtgewicht der Zusammensetzung, enthält.

23. Verfahren zum Färben von menschlichen Keratinfasern und insbesondere Haaren, **dadurch gekennzeichnet, dass** bei der Anwendung eine Farbmittelzusammensetzung, wie sie in einem der Ansprüche 1 bis 22 beschrieben ist, mit einer oxidierenden Zusammensetzung vermischt wird, das erhaltene Gemisch auf die Fasern aufgetragen und 3 bis 50 Minuten und vorzugsweise 5 bis 30 Minuten einwirken gelassen wird, worauf gespült, mit Haarwaschmittel gewaschen, nochmals gespült und getrocknet wird, wobei die oxidierende Zusammensetzung Wasserstoffperoxid oder eine Verbindung, die in situ Wasserstoffperoxid freisetzen kann, oder ein Redoxenzym enthält.

## Claims

1. Composition for the oxidation dyeing of human keratinous fibres and more particularly hair, comprising, in a cosmetically acceptable medium based on water, and at a basic pH, at least one oxidation dye and an alkalinizing agent, **characterized in that** the alkalinizing agent is a combination of at least one metasilicate chosen from the group consisting of alkali metal, alkaline-earth metal or ammonium metasilicates and at least one alkanolamine, with an anhydrous metasilicate/alkanolamine weight ratio of between 0.2 and 100.

2. Composition according to Claim 1, **characterized in that** the metasilicate is a sodium metasilicate.

3. Composition according to either of the preceding claims, **characterized in that** the alkanolamines are chosen from the group consisting of monoethanolamine, triethanolamine, monoisopropanolamine, diisopropanolamine, N-dimethylaminoethanolamine, 2-amino-2-methyl-1-propanol, triisopropanolamine, 2-amino-2-methyl-1,3-propanediol, 3-amino-1,2-propanediol, 3-dimethylamino-1,2-propanediol and trishydroxymethylaminomethane.

4. Composition according to Claim 3, **characterized in that** the alkanolamine is monoethanolamine.

5. Composition according to any one of the preceding claims, **characterized in that** the alkalinizing agent comprises, as active substance, from 0.1 to 6% by weight of metasilicate relative to the total weight of the composition.

6. Composition according to Claim 5, **characterized in that** the alkalinizing agent comprises, as active substance, from 0.5 to 5% by weight of metasilicate relative to the total weight of the composition.

7. Composition according to Claim 6, **characterized in that** the alkalinizing agent comprises, as active substance, from 1 to 3% by weight of metasilicate relative to the total weight of the composition.

8. Composition according to any one of the preceding claims, **characterized in that** the alkalinizing agent comprises, as active substance, from 0.1 to 8% by weight of alkanolamine relative to the total weight of the composition.

9. Composition according to Claim 8, **characterized in that** the alkalinizing agent comprises, as active substance, from 0.5 to 6% by weight of alkanolamine relative to the total weight of the composition.

10. Composition according to Claim 9, **characterized in that** the alkalinizing agent comprises, as active substance, from 1 to 5.5% by weight of alkanolamine relative to the total weight of the composition.

11. Composition according to any one of the preceding claims, **characterized in that** its pH is from 7.2 to 13.

12. Composition according to Claim 11, **characterized in that** its pH is from 8.5 to 11.5.

13. Composition according to any one of the preceding claims, **characterized in that** the oxidation dye or dyes are chosen from oxidation bases and couplers.

14. Composition according to Claim 13, **characterized in that** it comprises at least one oxidation base.

15. Composition according to Claim 14, **characterized in that** the oxidation bases are chosen from the group consisting of ortho- and para-phenylenediamines, double bases, ortho- and para-aminophenols, heterocyclic bases and their addition salts with an acid.

16. Composition according to Claim 13, **characterized in that** the couplers are chosen from the group consisting of meta-aminophenols, meta-phenylenediamines, meta-diphenols, naphthols, indole derivatives, indoline derivatives, sesamol and its derivatives, pyridine derivatives, pyrazolotriazole derivatives, pyrazolones, indazoles, benzimidazoles, benzothiazoles, benzoxazoles, 1,3-benzodioxoles, quinolines and their addition salts with an acid.

17. Composition according to any one of Claims 13 to 16, **characterized in that** the addition salts with an acid of the oxidation bases and couplers are chosen from the hydrochlorides, hydrobromides, sulphates, tartrates, lactates and acetates.

18. Composition according to any one of Claims 13-15 and 17, **characterized in that** the oxidation base or bases are present at a concentration ranging from 0.0005 to 12% by weight relative to the total weight of the composition.

19. Composition according to any one of Claims 13, 16 and 17, **characterized in that** the coupler or couplers are present at a concentration between 0.0001 and 10% by weight relative to the total weight of the composition.

20. Composition according to any one of the preceding claims, **characterized in that** the cosmetically acceptable medium based on water comprises at least one organic solvent.

21. Composition according to Claim 20, **characterized in that** the organic solvent or solvents are present in proportions ranging from 1 to 40% by weight relative to the total weight of the composition.

22. Composition according to any one of the preceding claims, **characterized in that** it contains at least one cationic polymer in the proportion of 0.05 to 10% by weight and at least one nonionic surfactant in the proportion of 0.1 to 20% by weight relative to the total weight of the composition.

23. Method for dyeing human keratinous fibres and in particular hair, **characterized in that**, at the time of use, a dyeing composition as described in any one of Claims 1 to 22 is mixed with an oxidizing composition, **in that** the mixture obtained is applied to the fibres, it is allowed to act for 3 to 50 minutes, preferably 5 to 30 minutes, 1 after which the fibres are rinsed, washed with shampoo, rinsed again and dried, the said oxidizing composition comprising hydrogen peroxide or a compound capable of releasing hydrogen peroxide in situ, or an oxidoreduction enzyzne.
